# EUROPEAN PATENT SPECIFICATION

(11) **EP 4 448 068 B1**
(45) Date of publication and mention of the grant of the patent: **05.11.2025**
(21) Application number: 22830702.1
(22) Date of filing: 05.12.2022
(51) Int. Cl.: A61M 16/00, A61B 5/11, A61B 5/00

(54) **CONTROLLING A HIGH FLOW NASAL THERAPY DEVICE**
STEUERUNG EINER NASALEN THERAPIEVORRICHTUNG MIT HOHEM DURCHFLUSS
COMMANDE D'UN DISPOSITIF DE THÉRAPIE PAR HAUT DÉBIT NASAL

(30) Priority: 16.12.2021 US 202163290596 P
(43) Date of publication of application: 23.10.2024
(73) Proprietor: Koninklijke Philips N.V., 5656 AG Eindhoven (NL)
(72) Inventor: BRANS, Harold Johannes Antonius, 5656 AG Eindhoven (NL); DELLIMORE, Kiran Hamilton J., 5656 AG Eindhoven (NL); BOU JAWDE, Samer, 5656 AG Eindhoven (NL); DE GRAAF, Pascal, 5656AG Eindhoven (NL)
(74) Representative: Philips Intellectual Property & Standards
(86) International application number: PCT/EP2022/084327
(87) International publication number: WO 2023/110469

(56) References cited:
- US-A1- 2007 208 269
- US-A1- 2018 071 469
- US-A1- 2018 104 426
- US-A1- 2020 001 038
- US-A1- 2021 113 797
- US-A1- 2021 361 899

## Description

### FIELD OF THE INVENTION

The present invention relates to the field of high flow nasal therapy (HFNT), and particularly to controlling a HFNT device.

### BACKGROUND OF THE INVENTION

High flow nasal therapy (HFNT) is a type of respiratory support method for subjects with hypoxemic respiratory failure. HFNT involves the delivery of up to 100% humidified and heated mixed air-oxygen at flow rates of up to 60 litres per minute. This is accomplished using a simple oxygen supply system comprising an air-oxygen blender, an active humidifier, a single heated inspiratory circuit, and a nasal cannula.

The many clinical benefits provided by HFNT include: small levels of positive end-expiratory pressure (PEEP); anatomical dead space wash out of CO2; maintenance of mucociliary clearance due to heated and humidified air; enhanced patient comfort; improved gas exchange; reduced work of breathing; and reduced right ventricular preload.

Typically, when a subject is ambulating or exercising, higher air flow and oxygen concentrations (FiO2) are needed than when they are resting or stationary. In other words, a subject's HFNT needs change significantly according to their activity level. Ensuring HFNT settings are appropriate for the subject as they go about their day-to-day lives is therefore key to maximising subject comfort, and effectiveness of the therapy.

US 2018/104426 A1 describes an apparatus for oxygenation and/or CO2 clearance of a patient, comprising a flow source or a connection for a flow source.

US 2018/071469 A1 describes a method for controlling gases delivery to a patient, such as a via a patient interface.

US 2021/113797 A1 describes a control unit for use with a respiratory assist device that assists a subject with respiration.

US 2020/001038 A1 describes a pressure support system for providing pressure support therapy to a patient.

### SUMMARY OF THE INVENTION

The invention is defined by the claims.

According to an aspect of the invention, there is provided a control system for a high flow nasal therapy (HFNT) device used by a subject according to claim 1.

In this way, data relating to both physiological (i.e. corporal or physical body) parameters, and non-physiological (i.e. movement or activity) parameters may be leveraged in order to control a HFNT device (via a control signal). As such, HFNT settings may be more appropriate for the current, and potentially future, condition of the subject. Ultimately, this may improve the comfort of the subject and the overall effectiveness of the therapy.

Typical HFNT devices supply air at a constant air flow rate, and oxygen concentration level, which may be manually adjusted. The present invention uses information that may be acquired from physiological sensors and non-physiological sensors, to automatically, dynamically and seamlessly adjust the control of a HFNT device through a control signal. Indeed, the control system be used to generate a control signal for existing HFNT devices, obviating the need for the design of completely new HFNT devices with this capability.

Physiological sensors, such as blood oxygen saturation sensor SpO2, may be able to measure the state/condition of the subject, and therefore may provide an indication as to the activity level of the subject (and thus, subject-specific HFNT needs). However, non-physiological sensors that provide movement parameters may be leveraged in order to provide a more detailed pictures as to the activity level of the subject. Indeed, some movement parameter values indicate a level of activity, and the physiological parameter values may then be compared to the level of activity to determine whether current HFNT device parameters appear to be adequate (and thus inform a control signal for the HFNT device).

By way of example, an elevated subject heart rate may not necessarily imply that the HFNT device needs to be controlled differently. In the case that the subject is exercising more intensively than their baseline activity (as determined by a detected movement parameter), an elevated heart rate is normal. However, if the movement parameter indicates that the subject is merely lightly exercising, an elevated heart rate beyond a certain subject personal level may indicate that the HFNT devices needs to be controlled differently (i.e. increase a flow rate of air from the HFNT device. Thus, both physiological and movement parameters are necessary in order to obtain a complete picture of how well the HFNT device as it is currently operating is meeting subject needs.

Moreover, it is often the case that a subject movement/activity level will change before physiological parameter values (i.e. heart rate, SpO2) change, as there is a delay in the reaction time of the organs of the body. Thus, the proposed control system may be able to generate a control signal for the HFNT device that essentially anticipates subject needs.

Put another way, provided are concepts for controlling a high flow nasal therapy (HFNT) device used by a subject. In particular, physiological and movement parameter values of the subject are leveraged in order to generate a control signal for the HFNT device. These parameters may indicate an activity level of the subject, as well as the condition of the subject, providing information useful for setting appropriate operating conditions of the HFNT device. Thus, a means for automatically controlling a HFNT device based on needs of the subject may be provided, improving subject comfort during therapy, and ease of use of the HFT device.

In some embodiments, the control unit may be configured to: determine a target HFNT parameter value based on the detected physiological parameter value and movement parameter value; and generate the control signal based on the target HFNT parameter value.

In some embodiments, the control system may further comprise a HFNT device interface unit configured to receive an operating parameter value of the HFNT device, and the control unit may be configured to generate the control signal further based on the operating parameter value.

In this way, it may be fully understood how the subject and HFNT device are interacting. This may provide a closed feedback loop, such that a control signal is generated that may control the HFNT device in an effective and efficient manner. A closed feedback loop may ensure that an equilibrium is found between subject needs, and the air that the HFNT device may supply.

In some embodiments, the control system may further comprise a wearable device, wherein the wearable device comprises at least one of the physiological sensor unit and the activity sensor unit.

In other words, at least one (if not both) of the physiological and non-physiological sensor units may be supplied within a wearable device (i.e. a watch, bracelet, necklace, etc.), or a combination of wearable devices. Alternatively, the sensor unit may be supplied in separate wearable devices, or another portable device. This improves the ease of use of the control system for the subject as they go about their day-to-day activities.

In some embodiments, the control signal may be configured for controlling at least one of a flow rate and an oxygen concentration of air provided by the HFNT device.

By way of explanation, two key parameters of HFNT are the flow rate and the oxygen concentration. The flow rate is the volume of air supplied by the HFNT device to the subject (i.e. through the nasal cannula) in a given amount of time. The oxygen concentration is the percentage of air supplied to the subject that is oxygen, and is often known as the fraction of inspired oxygen (FiO2). Thus, the control signal may control at least these two parameters in order to ensure appropriate settings for HFNT to improve subject comfort and effectiveness of therapy.

In some embodiments, the detected physiological parameter may include at least one of: an oxygen saturation; a heart rate; a heart rate variability; a respiration rate; respiration rate variability; a galvanic skin response; and a transcutaneous CO2 value.

Specifically, oxygen saturation (SpO2) provides useful information regarding the effectiveness of current HFNT. If oxygen saturation drops below a certain level, this provides a good indicator that HFNT settings much be changed (i.e. increase a flow, or oxygen concentration of air supplied by the HFNT device). However, each of these parameters provide useful information regarding the state of the subject, and therefore may be useful for generating an appropriate control signal. It should be understood that this is not an exhaustive list, and other physiological parameters indicative of a state/condition of the subject may be used, as understood by the skilled person.

In some embodiments, the detected movement parameter may include at least one of: an acceleration; a velocity; a displacement; an inclination; and a change of position.

Each of these parameters supply a clue/hint/information useful for determining the activity level and circumstances of the subject. Indeed, each of these may be acquired by a gyroscope and accelerometer, which are usually present many smart-devices. Overall, by detecting at least one of these parameters, an appropriate control signal for the HFNT device may be generated. It should be understood that this is not an exhaustive list, and other parameters indicative of an activity/motion/movement of the subject may be used, as understood by the skilled person.

The control unit further comprises an activity classification component configured to classify the subject into one of a plurality of activity classifications based on the movement parameter value, and generating the control signal is further based on the activity classification of the subject.

Indeed, the movement parameter value is used to classify/identify the activity of the subject. The activity classification may be an activity level, or may be a specific activity (e.g. treadmill running, stair climbing, etc.). Using this knowledge, an appropriate control signal is generated for the HFNT device.

In some embodiments, the control system may further comprise an environmental sensor unit configured to detect an ambient environment parameter value of the environment surrounding the subject, and the control unit may be configured to generate the control signal further based on the ambient parameter value.

In some embodiments, the detected ambient environment parameter may include at least one of: a temperature; a humidity; an air pressure; a particle concentration value; and an air quality value.

As well as the current status/condition/activity level of the subject, the surroundings/ambient conditions of the subject also impact the control of the HFNT device required to meet subject needs. For example, a higher flow rate and/or oxygen concentration is required in a hot/humid/low air quality area, compared to a cold/dry/high air quality area. Thus, this may provide further information useful in generating an appropriate control signal for the HFNT device.

In some embodiments, the control system may further comprise an information acquisition unit configured to obtain subject-specific historical data describing a previously determined parameter value for the subject, and the control unit may be configured to generate the control signal further based on the subject-specific historical data.

In some embodiments, the subject-specific historical data may include at least one of: historical HFNT operating parameters associated with the subject; and clinical data of the subject.

In some embodiments, the clinical data of the subject may include at least one of: an age; a height; a weight; a BMI; medical conditions; respiratory conditions; an exposure to air pollution; and a smoking history.

Indeed, information regarding the subject may allow for more appropriate HFNT setting values (by the generated control signal). For example, subjects who are older, with severe pre-existing medical conditions may require a higher flow and/or oxygen concentration than subjects who are young, with minor pre-exiting conditions.

Moreover, historical HFNT operating parameters may be used to inform current desired HFNT operating parameters (and thus the control signal to meet such desired HFNT operating parameters). For example, if previously the subject having the same/similar movement parameters and physiological parameters benefitted from a certain control signal being generated, then this signal generation may occur again.

In some embodiments, the control unit may be configured to generate the control signal responsive to at least one of: a predetermined amount of time elapsing since generating a previous control signal; a change in the detected physiological parameter value exceeding a first predetermined threshold; and a change in the detected movement parameter value exceeding a second predetermined threshold.

In this way, the control unit may generate the control signal at appropriate moments, such that the HFNT device is controlled to have appropriate parameter values at all times (without unnecessary constant recalculation of the control signal.

According to yet another aspect of the invention, there is provided a computer program comprising computer program code means adapted, when said computer program is run on the system of claim 1, to implement a method for controlling a HFNT device used by a subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates changing subject parameter values, along with a changing target high flow nasal therapy (HFNT) parameter value, as a subject activity level changes;
Fig. 2 is a simplified block diagram of a control system for a HFNT device used by a subject according to an exemplary embodiment; and
Fig. 3 is a flow diagram of a method for controlling a HFNT device used by a subject which is not claimed

### DETAILED DESCRIPTION OF THE EMBODIMENTS

The invention will be described with reference to the Figures.

It should be understood that the detailed description and specific examples, while indicating exemplary embodiments of the system and computer program are intended for purposes of illustration only and are not intended to limit the scope of the invention. These and other features, aspects, and advantages of the system and computer program of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to".

It should be understood that the Figures are merely schematic and are not drawn to scale. It should also be understood that the same reference numerals are used throughout the Figures to indicate the same or similar parts.

According to proposed concepts, a number of possible solutions may be implemented separately or jointly. That is, although these possible solutions may be described below separately, two or more of these possible solutions may be implemented in one combination or another.

Embodiments of the invention aim to provide concepts for controlling a HFNT device used by a subject. In particular, data from physiological and non-physiological sensors is leveraged in order to generate a control signal for the HFNT device. In this way, control off the HFNT device may be achieved based on real-time needs of the subject. For example, HFNT parameter values may be changed if the subject is exercising, thus improving the comfort of the subject.

Put another way, there is a present need for a means to generate a control signal for HFNT devices, such that the HFNT device may react to a subject state/condition/activity level. In this way, the HFNT device may be controlled appropriately, for effective therapy of the subject, resulting in improved subject comfort. This may be achieved by the acquisition of physiological and movement parameters by sensor units, which may indicate the current state/condition/activity level, and may provide clues as to the future state/condition/activity level of the subject. Indeed, as it is now increasingly typical to use wearable-smart devices including such sensors, embodiments of the invention provide a simple, non-invasive means for controlling HFNT devices using existing hardware.

Embodiments of the present invention address the problem of inadequate and sub-optimal adaptation of HFNT settings to meet a subject's varying flow and oxygen concentration (FiO2) demands as they go about their day-to-day lives. **In** particular, the challenge of smooth adaptation of the HFNT flow and oxygen delivery as the subject transitions between periods of different physical activity is addressed. Moreover, adaption is not restricted only between physical activities, but can also be used during a physical activity.

Moreover, embodiments of the invention provide a control system for a HFNT device of the subject may be supplied without the need for any architectural or functional changes to the HFNT system. In other words, existing HFNT systems may be used alongside embodiments of the invention without modification, thus providing control of HFNT parameters based on subject measurements.

The control system improves HFNT delivery to the subject, responding to changes in the subject condition (and environment). Thus, efficient use of oxygen is made, whilst also ensuring the subject is receiving effective therapy.

Moreover, by providing a means by which HFNT may be adapted in response to changes in a subject state, a comfort level of the subject may be improved. Indeed, the subject may not need to worry about altering settings manually in response to changes in their comfort levels, with embodiments of the control system proactively altering HFNT parameters to ensure subject comfort regardless of the circumstances.

According to some embodiments of the invention, the following features are provided to obtain the above advantages:
(i) A wearable or body-worn unit with one or more inertial sensors (e.g. an accelerometer, a gyroscope) which semi-continuously acquire non-physiological (movement) measurements from a subject, including acceleration, velocity, displacement, motion, inclination and change of position;
(ii) A wearable or body-worn unit with one or more sensors (e.g., PPG, GSR etc.) which semi-continuously acquires physiological measurements from a subject, including at least one of SpO2, heart rate (HR), heart rate variability (HRV), respiration rate (RR), respiration rate variability (RRV) and/or galvanic skin response (GSR);
(iii) A processing unit containing a CPU which processes the acquired measurements and determines a prediction of the subject's flow and oxygen needs at a given moment in time; and
(iv) A control unit which adapts the HFNT flow and oxygen (FiO2) settings based on receiving input on the subject's current flow and oxygen needs.

Further, other embodiments of the invention, the control system may also comprise:
(v) One or more ambient/environmental sensors in the vicinity of the subject which acquire contextual information about the subject which may influence the subject's HFNT flow and oxygen delivery needs. This may include, for example, information regarding the ambient temperature, humidity, and air quality (i.e., ozone (O3), sulphur dioxide (SO2), nitrogen dioxide (NO2), carbon monoxide (CO), particles smaller than 2.5 micrometers (PM2.5), and particles smaller than 10 micrometers (PM10) etc.).

By way of explanation, embodiments of the invention (semi) continuously measure both movement/activity/non-physiological parameters and physiological parameters. This information is then analysed by a control unit to generate a control signal for the HFNT device. Specifically, oxygen and flow needs of the subject may be predicted, which are in turn used by the control unit to automatically and dynamically adjust the HFNT settings via the control signal.

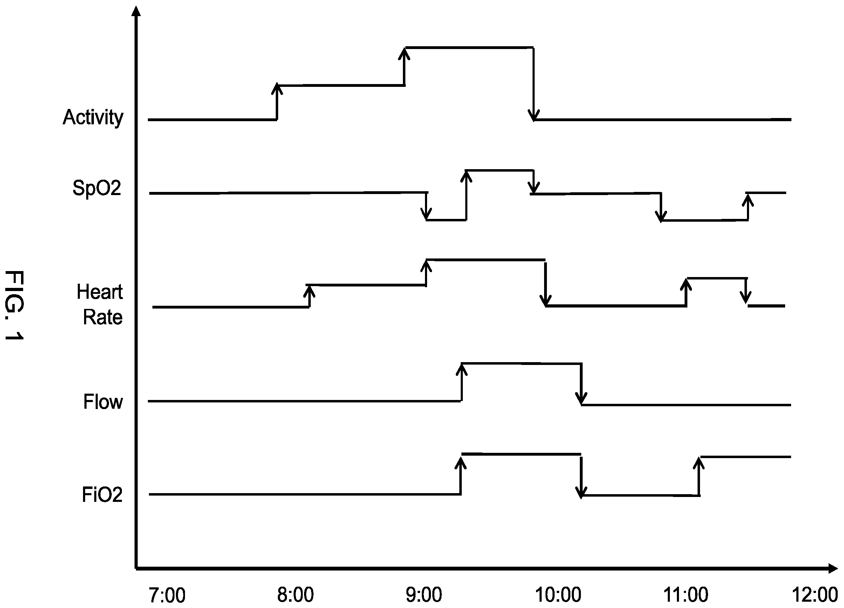

Fig. 1 also demonstrates how the control system may form a closed feedback loop. In particular, historical data (both clinical data of the subject and historical HFNT operating parameters), can help in generating the control signal to calibrate the device settings to a personal level. The response of the subject to the adaption of the HFNT settings may also be measured by the wearable sensors. In this way, a closed feedback loop is established, leading to a stable state of appropriate HFNT control values.

More specifically, movement parameters that may be of relevance to HFNT control include an acceleration, velocity, displacement, motion, inclination and/or change of position of the subject. These values may be easily captured using a wearable patch or smartwatch equipped with an inertial sensor (i.e., accelerometer and or gyroscope).

By way of example, when the subject is moving for long periods of time (e.g., > 1 min or 2 min) or they are moving at a certain velocity (e.g., > 1.4 m/s) or acceleration (>0.5 ms⁻²), or they are going up or down stairs (as determined from the inclination of the accelerometer or gyroscope), the control signal may be generated to increase the flow rate and/or oxygen concentration (FiO2) supplied by the HFNT device. For instance, the flow rate may increase from 10 L/min to 30 L/min and the FiO2 from 0.21 to 1.00. Conversely, when the subject becomes stationary, (e.g. sitting or lying down) the control signal may be generated such that the flow rate and oxygen concentration may be decreased, since the subject's needs are reduced.

Moreover, the magnitude of increase or decrease in the flow rate and oxygen concentration may be correlated with the intensity of the activity being performed by the subject. The flow rate and oxygen concentration may also be ramped up or down gradually. For example, if the subject climbs a small flight of stairs (e.g., 5-10 steps) only an increased flow rate from say 10 to 30 L/min may be needed to maintain the subject's comfort, while if the subject climbs a large flight of stairs (e.g., >50 steps) the flow rate may be increased first from 10 L/min to 30 L/min and the FiO2 may be increased from 0.21 to 0.5, and later there may be a further increase in flow rate ad FiO2 to 40 L/min and 0.75, respectively.

In a further embodiment, movement parameter values acquired by the non-physiological sensor unit are also used to classify the activity being performed by the subject. In this way, the generated control signal may be further fine-tuned to suit the subject's needs. For example, if the patient is cycling or training with gym equipment (e.g., a stair climber, elliptical machine or stationary bike) or playing a sport (e.g., golf or table-tennis), this information can be used to set suitable flow rate and oxygen concentration settings which ensure appropriate HFNT (and thus subject comfort).

Moving on, physiological parameters that may be of relevance to HFNT control include SpO2, RR, HRV, RR, RRV and GSR. These parameter values may be acquiring using a wearable patch or smart watch, or by using a body-worn unit such as the nasal alar sensor.

By measuring physiological parameters, a subject's flow rate and oxygen concentration needs can be directly assessed allowing for a more appropriate adjustment of the HFNT device settings by the generated control signal.

For example, if a subject's SpO2 level falls (i.e. from 95% to 92%) the control unit may trigger an increase in the FiO2 delivered (i.e. from 0.21 to 0.50), while also increasing the flow rate (i.e. from 10 L/min to 30 L/min). By measuring physiological parameters, we are better able to track the changing oxygen and flow needs of the patient, and dynamically adjust HFNT settings.

Although SpO2 is the most advantageous physiological parameter to measure in order to determine needs of a subject, other vital signs may also be useful to measure, as well since they may respond more quickly to changes in the patient's condition. For example, changes in HR and RR are useful indicators of the intensity of physical activity. In addition, HRV and RRV are indicators of subject stress and anxiety, which may significantly impact the subject's HFNT flow and oxygen concentration needs. Moreover, GSR is a useful measure of intensity of physical activity (based on the sweat rate - higher sweat rates are associated with more intense physical activity) and of stress/anxiety. So, GSR may be used as an alternative to HRV or RRV or for confirmation of the patient stress level derived from HRV and RRV.

Furthermore, ambient/environmental conditions, in the vicinity of the subject may significantly influence the oxygen and flow rate needs of the subject. If a subject is in a hot, humid climate they will have higher oxygen and flow rate needs than when they are in a mild, moderate climate. The same also applies for circumstances in which the subject is exposed to poor air quality, such as a place with high amounts of air pollution and smog, as well as places with high pollen count. In a home environment, air pollution can be present during cooking, and by using deodorants and perfumes.

Thus, according to yet further embodiments of the invention, information obtained may be obtained from an environmental sensor unit configured to detect ambient environment parameter values. These values may be used alongside the movement and physiological parameter values to generate the control signal, and consequently further refine the optimization of HFNT settings for the subject. This includes measurements of room temperature, humidity, air quality (e.g. ozone (O3), sulphur dioxide (SO2), nitrogen dioxide (NO2), carbon monoxide (CO), volatile organic compounds (VOC), etc.) and particle concentration (e.g. particles smaller than 2.5 micrometres (PM2.5), particles smaller than 10 micrometers (PM10), ultrafine particles (UFP), pollen count, etc.).

Fig. 1 exemplifies how the measured movement and physiological parameters may be used to control a HFNT of the subject during various stages of a typical day. Specifically, it is shown how physciological parameters SpO2 and HR are used alongside an activity classification (determined by movement parameters), in order to control a flow rate and an oxygen concentration of the HFNT device.

Initially, the subject may is reading newspapers from 7:00 to 8:00. During this rest time, the HFNT device settings may be baseline settings, set by a clinician. Alternatively, these settings may be automatically titrated by the control device such that normal ranges of SpO2 are obtained at resting heart rate.

At 8:00, the subject initiates some light exercise (i.e. walking). As the level of activity of the subject increases, the heart rate of the subject increases. In this case however, SpO2 level remains normal and thus it is determined that the subject's own cardiorespiratory systems can provide the required oxygen levels (adequate SpO2). In this case, the flow of the device and the FiO2 could be kept the same (as shown), or may be slightly increased a control signal to the HFNT device in order to provide a small level of support.

At 9:00, the subject begins more intense exercise (i.e. use of a treadmill) leading to more activity. Thus, the subject's heart rate further increases, and SpO2 decreases. Therefore, it is clear that SpO2 levels cannot be compensated by the subject without support by HFNT. After a short period, a control sign adjusts the HFNT device settings (both flow and FiO2) such that SpO2 returns to normal (and perhaps even reduces the subject's heart rate). However, heart rate is not expected to go back to baseline because the patient is exercising (as determined by the activity monitor).

At 10:00, the subject rests and the settings could go back to baseline to conserve power and oxygen. At 11:00 air pollutants may cause a decrease in the subject's SpO2 level. In this case, because the activity level is at baseline, it is understood that increase in heart rate is not because of exercise but because the body is trying to compensate reduced oxygen blood levels. Thus, FiO2 is adjusted to return both heart rate and SpO2 to normal.

It should be understood that the adaption of air flow and FiO2 by a control signal is not restricted only between physical activities, but can also be used during a (physical) activity. Due to fact that the measurements may be performed quasi-continuously, fine-tuning of the generated control signal may be performed as often as needed. For example, if the SpO2 level of the subject does not rise as much as predicted at an increased level of flow and/or FiO2, a further increase may be implemented.

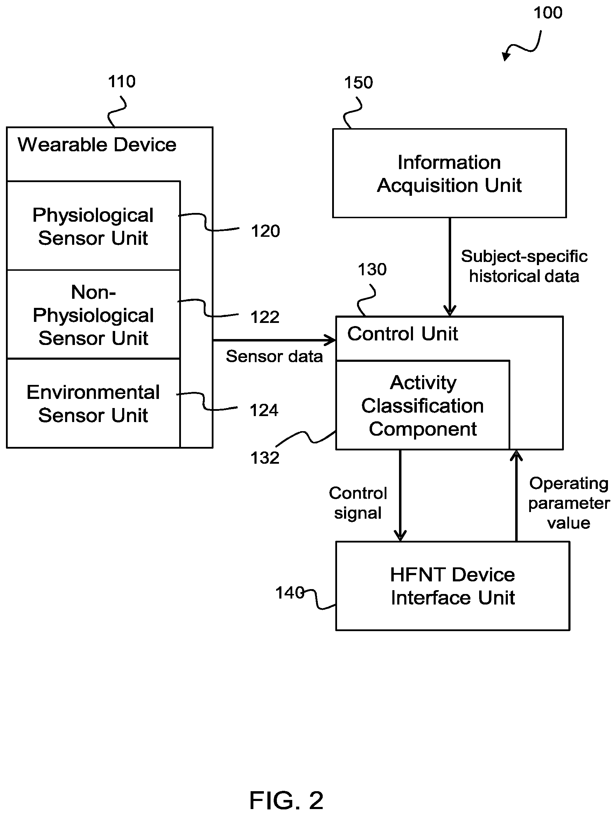

Fig. 2 presents a simplified block diagram of a control system 100 for a HFNT device used by a subject according to an exemplary embodiment. Specifically, the control system 100 comprises a physiological sensor unit 120, a non-physiological sensor unit 122, and a control unit 130 and an activity classification component 132. Optionally, the control system 100 may also comprise an environmental sensor unit 124, an information acquisition unit 150, and a HFNT device interface unit 140.

The physiological sensor unit 120 is configured to detect a physiological parameter value of the subject. The physiological sensor unit 120 may include an arrangement of one or more sensors configured to detect/measure/ascertain physiological values of a subject. For example, the physiological sensor unit 120 may include a pulse oximeter, a pulse sensor, a respiratory rate sensor, or any other sensor suitable for measuring vital signs known in the art. Physiological parameters may be any measurable parameter related to the function of a subject's body (i.e. vital signs).

More specifically, the detected physiological parameter may include one or more of: an oxygen saturation (i.e. a blood oxygen saturation level); a heart rate (i.e. beats per minute); a heart rate variability (i.e. variation in the beat-to-beat interval); a respiration rate (i.e. breaths per minute); respiration rate variability (i.e. variation in the breath-to-breath interval); a galvanic skin response (i.e. changes in sweat gland activity); and a transcutaneous CO2 value (i.e. arterial CO2 concentration).

The non-physiological sensor unit 122 is configured to detect a movement parameter value of the subject. The non-physiological sensor unit 122 includes an arrangement of one or more sensors configured to detect/measure/ascertain movement values of a subject. For example, the non-physiological sensor unit 122 may include an accelerometer, a gyroscope, or any other sensor suitable for measuring movement of the subject known in the art. Movement parameters may be any measurable parameter related to the movement/activity/motion of the subject.

In particular, the detected movement parameter may include one or more of: an acceleration; a velocity; a displacement; an inclination; an altitude; and a change of position.

Optionally, an environmental sensor unit 124 may be included in the control system 100. The environmental sensor unit 124 is configured to detect an ambient environment parameter value of the environment surrounding the subject. In other words, the environmental sensor unit 124 may comprise an arrangement of one or more sensors suitable for detecting/measuring/ascertaining the ambient conditions in the area surrounding the subject. For example, the environmental sensor unit 124 may include a thermometer, humidity sensor, a barometer, an air quality monitor, or any other sensor suitable for measuring environmental conditions known in the art. Environmental parameters may be any measurable parameter related to the surrounding environment of the subject.

Specifically, the detected ambient environment parameter may include one or more of: a temperature; a humidity; an air pressure; a particle concentration value (e.g. number of particles smaller than 2.5 micrometres (PM2.5), and particles smaller than 10 micrometres (PM10), ultrafine particles (UFP), and a pollen count); and an air quality value (e.g. concentration of ozone (O3), sulphur dioxide (SO2), nitrogen dioxide (NO2), carbon monoxide (CO), and volatile organic compounds (VOC) in the air).

The control system 100 may further comprise a wearable device 110. The wearable device 110 comprises one or more of the physiological sensor unit 120, the non-physiological sensor unit 122, and the environmental sensor unit 124 (if included). In this way, the sensor units may be kept proximate to the subject, ensuring accurate measurements. The wearable device 110 may be any portable device suitable for strapping, clipping, or otherwise attaching to the subject. For example, the wearable device 110 may be a watch, a bracelet, a necklace, a sweatband, etc).

Indeed, each of the sensor units may be integrated with a different wearable device 110, communicatively connected to each other and the control unit 130. The control unit 130 may also be integrated/provided on a wearable device 110, or may be provided externally (i.e. on a laptop, smartphone, etc.). In any case, the sensor units are communicatively connected (directly, or indirectly) to the control unit 130 (i.e. wired connect, Bluetooth, Wi-Fi, etc.), such that the parameter values are transmitted to the control unit 130.

Moving on, the control unit 130 is configured to generate a control signal for the HFNT device based on the physiological parameter value and the movement parameter value. In other words, the control unit 130 is configured to receive the physiological parameter value and the movement parameter value from the sensor units, and determine a control signal for the HFNT device based on processing the received parameter values.

The control signal may be any signal suitable for controlling the operating parameters of the HFNT device. The control signal may merely be a relative signal (i.e. increase/decrease a parameter), or may be an absolute signal (i.e. change the parameter to a specific amount). In any case, the control signal may be adapted to be suitable for the HFNT device that is being controlled.

In some cases, the control signal comprises information for controlling at least one of a flow rate (i.e. the volume of air per minute output by the nasal cannula of the HFNT device) and an oxygen concentration (i.e. percentage of air that is oxygen) of air provided by the HFNT device. For example, the control signal may provide a command to increase the flow rate to a certain value, or to simply increase the flow rate by an amount.

Indeed, in the case that the environmental sensor unit 124 is provided, the control unit 130 is configured to generate the control signal further based on the ambient parameter value. In other words, the control unit 130 will take the movement parameter, the physiological parameter and the ambient parameter value into account when generating the control signal.

More specifically, the control unit 130 may be configured to determine/calculate/ascertain a target HFNT parameter value based on the detected physiological parameter value and movement parameter value (i.e. an oxygen concentration of 30%). The control unit 130 may then be configured to generate/produce the control signal based on the target HFNT parameter value.

Moreover, the control unit 130 may be configured to generate the control signal responsive to one or more trigger conditions. The trigger condition may include a predetermined amount of time elapsing since generating a previous control signal, a change in the detected physiological parameter value exceeding a first predetermined threshold, and/or a change in the detected movement parameter value exceeding a second predetermined threshold.

**In** other words, the control unit 130 may generate the control signal at regular time intervals, or when a change in the subject condition or movement exceeds a certain threshold. **In** this way, the control signal may remain up-to-date, without an unnecessary excess of calculations.

**In** some embodiments, the control system 100 may further comprise an information acquisition unit 150. The information acquisition unit 150 is configured to obtain subject-specific historical data describing a previously determined parameter value for the subject, and wherein the control unit 130 is configured to generate the control signal further based on the subject-specific historical data.

The information acquisition unit 150 may obtain the subject information from memory, from external databases (such as electronic medical records), or from data saved by the control system.

**In** other words, the information acquisition unit 150 may be provided to obtain information related to previous movement, physiological and/or environmental parameters. Indeed, information from these circumstances regarding control signals may also be included. This previous information may prove useful for determining/generating an appropriate control signal.

Indeed, the subject-specific historical data may include one, or both, of historical HFNT operating parameters associated with the subject, and clinical data of the subject. The clinical data of the subject may include one or more of: an age; a height; a weight; a BMI; medical conditions; respiratory conditions; an exposure to air pollution; and a smoking history. Indeed, the data is not restricted this, and may include any historical data relating to the subject that may be relevant for determining an appropriate control signal for the HFNT device.

The control unit 130 includes an activity classification component 132. The activity classification component 132 is configured to classify the subject into one of a plurality of activity classifications based on the movement parameter value, and wherein generating the control signal is further based on the activity classification of the subject.

Put another way, the activity classification component 132 uses the movement parameter value to determine an activity classification of the subject. The activity classification may, for example, designate an activity level (i.e. low, medium, high), and/or an activity type (i.e. running, climbing, cycling, etc.).

Finally, the control system 100 may further comprise a HFNT device interface unit 140. The HFNT device interface unit 140 may be configured to receive an operating parameter value of the HFNT device (i.e. from the HFNT device). Indeed, the HFNT device interface unit 140 may also be configured to receive the control signal, and thus control the HFNT device based on the control signal.

In this case, the control unit 130 is configured to generate the control signal further based on the operating parameter value (along with the movement parameter, the physiological parameter, and any other optional information).

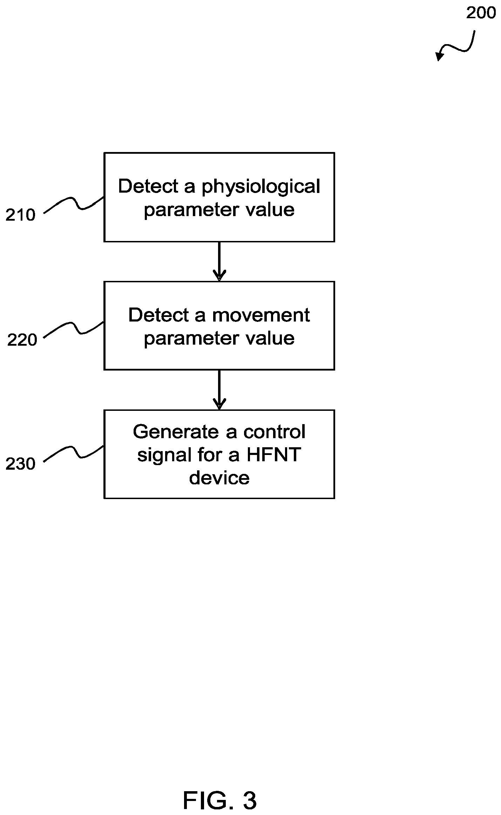

Fig. 3 presents a flow diagram of a method 200 for controlling a HFNT device used by a subject which is not claimed.

At step 210, a physiological parameter value of the subject is detected/measured. At step 220, a movement parameter value of the subject is detected/measured. Indeed, both the movement parameter and the physiological parameter may be (semi) continuously detected, and may be simultaneously. Thus, a constant monitoring of the physiology and activity of the subject may be performed.

At step 230, a control signal for the HFNT device is generated, based on the physiological parameter value and the movement parameter value. Thus, the HFNT device may be controlled based on the activity and physiological condition of the subject, improving the appropriateness of the therapy provided by HFNT.

A single processor or other unit may fulfil the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage.

Any reference signs in the claims should not be construed as limiting the scope.

## Claims

1. A control system (100) for a high flow nasal therapy, HFNT, device used by a subject, the system comprising:
a physiological sensor unit (120) configured to detect a physiological parameter value of the subject;
a movement sensor unit (122) configured to detect a movement parameter value of the subject indicating an activity level of the subject;
an activity classification component configured to classify the subject into one of a plurality of activity classifications based on the movement parameter value; and
a control unit (130) configured to generate a control signal for the HFNT device based on the activity classification of the subject, the physiological parameter value and the movement parameter value.

2. The control system of claim 1, wherein the control unit (130) is configured to:
determine a target HFNT parameter value based on the detected physiological parameter value and movement parameter value; and
generate the control signal based on the target HFNT parameter value.

3. The control system of claim 1 or 2, further comprising a HFNT device interface unit (140) configured to receive an operating parameter value of the HFNT device, and wherein the control unit (130) is configured to generate the control signal further based on the operating parameter value.

4. The control system of any of claims 1 to 3, further comprising a wearable device (110), and wherein the wearable device comprises at least one of the physiological sensor unit (120) and the movement sensor unit (124).

5. The control system of any of claims 1 to 4, wherein the control signal is configured for controlling at least one of a flow rate and an oxygen concentration of air provided by the HFNT device.

6. The control system of any of claims 1 to 5, wherein the detected physiological parameter includes at least one of:
an oxygen saturation;
a heart rate;
a heart rate variability;
a respiration rate;
respiration rate variability;
a galvanic skin response; and
a transcutaneous CO2 value.

7. The control system of any of claims 1 to 6, wherein the detected movement parameter includes at least one of:
an acceleration;
a velocity;
a displacement;
an inclination; and
a change of position.

8. The control system of any of claims 1 to 7, further comprising an environmental sensor unit (124) configured to detect an ambient environment parameter value of the environment surrounding the subject, and wherein the control unit (130) is configured to generate the control signal further based on the ambient parameter value.

9. The control system of claim 8, wherein the detected ambient environment parameter includes at least one of: a temperature; a humidity; an air pressure; a particle concentration value; and an air quality value.

10. The control system of any of claims 1 to 9, further comprising an information acquisition unit (150) configured to obtain subject-specific historical data describing a previously determined parameter value for the subject, and wherein the control unit (130) is configured to generate the control signal further based on the subject-specific historical data.

11. The control system of claim 10, wherein the subject-specific historical data includes at least one of:
historical HFNT operating parameters associated with the subject; and
clinical data of the subject,
and preferably wherein the clinical data of the subject includes at least one of: an age; a height; a weight; a BMI; medical conditions; respiratory conditions; an exposure to air pollution; and a smoking history.

12. The control system of any of claims 1 to 11, wherein the control unit (130) is configured to generate the control signal responsive to at least one of:
a predetermined amount of time elapsing since generating a previous control signal;
a change in the detected physiological parameter value exceeding a first predetermined threshold; and
a change in the detected movement parameter value exceeding a second predetermined threshold.

13. A computer program comprising computer program code means adapted, when said computer program is run on a control system according to claim 1, to implement a method (200) comprising:
detecting (210) a physiological parameter value of the subject;
detecting (220) a movement parameter value of the subject indicating an activity level of the subject;
classifying the subject into one of a plurality of activity classifications based on the movement parameter value; and
generating (230) a control signal for the HFNT device based on the activity classification of the subject, the physiological parameter value and the movement parameter value.

## Patentansprüche

1. Steuersystem (100) für eine von einem Subjekt verwendete Vorrichtung zur nasalen Therapie mit hohem Durchfluss, HFNT, wobei das System umfasst:
eine physiologische Sensoreinheit (120), welche dazu konfiguriert ist, einen physiologischen Parameterwert des Subjekts zu erfassen;
eine Bewegungssensoreinheit (122), welche dazu konfiguriert ist, einen Bewegungsparameterwert des Subjekts zu erfassen, welcher ein Aktivitätsniveau des Subjekts angibt;
eine Aktivitätsklassifizierungskomponente, welche dazu konfiguriert ist, das Subjekt basierend auf dem Bewegungsparameterwert in eine von einer Vielzahl von Aktivitätsklassifizierungen zu klassifizieren; und
eine Steuereinheit (130), welche dazu konfiguriert ist, basierend auf der Aktivitätsklassifizierung des Subjekts, dem physiologischen Parameterwert und dem Bewegungsparameterwert ein Steuersignal für die HFNT-Vorrichtung zu erzeugen.

2. Steuersystem nach Anspruch 1, wobei die Steuereinheit (130) dazu konfiguriert ist:
einen HFNT-Zielparameterwert basierend auf dem erfassten physiologischen Parameterwert und Bewegungsparameterwert zu bestimmen; und
das Steuersignal basierend auf dem HFNT-Zielparameterwert zu erzeugen.

3. Steuersystem nach Anspruch 1 oder 2, weiter eine HFNT-Vorrichtungsschnittstelleneinheit (140) umfassend, welche zum Empfangen eines Betriebsparameterwerts der HFNT-Vorrichtung konfiguriert ist, und wobei die Steuereinheit (130) dazu konfiguriert ist, das Steuersignal weiter basierend auf dem Betriebsparameterwert zu erzeugen.

4. Steuersystem nach einem der Ansprüche 1 bis 3, weiter eine tragbare Vorrichtung (110) umfassend, und wobei die tragbare Vorrichtung zumindest eine von der physiologischen Sensoreinheit (120) und der Bewegungssensoreinheit (124) umfasst.

5. Steuersystem nach einem der Ansprüche 1 bis 4, wobei das Steuersignal zum Steuern von zumindest einer von einer Durchflussrate und einer Sauerstoffkonzentration von Luft, die von der HFNT-Vorrichtung bereitgestellt wird, konfiguriert ist.

6. Steuersystem nach einem der Ansprüche 1 bis 5, wobei der erfasste physiologische Parameter zumindest eines der folgenden beinhaltet:
eine Sauerstoffsättigung;
eine Herzfrequenz;
eine Herzfrequenzvariabilität;
eine Atemfrequenz;
Atemfrequenzvariabilität;
eine galvanische Hautreaktion; und
einen transkutanen CO2-Wert.

7. Steuersystem nach einem der Ansprüche 1 bis 6, wobei der erfasste Bewegungsparameter zumindest eines der folgenden beinhaltet:
eine Beschleunigung;
eine Geschwindigkeit;
eine Verschiebung;
eine Neigung; und
einen Positionswechsel.

8. Steuersystem nach einem der Ansprüche 1 bis 7, weiter eine Umfeldsensoreinheit (124) umfassend, welche dazu konfiguriert ist, einen Umgebungsumfeldparameterwert des das Subjekt umgebenden Umfelds zu erfassen, und wobei die Steuereinheit (130) dazu konfiguriert ist, das Steuersignal weiter basierend auf dem Umgebungsparameterwert zu erzeugen.

9. Steuersystem nach Anspruch 8, wobei der erfasste Umgebungsumfeldparameter zumindest eines der folgenden beinhaltet: eine Temperatur, eine Luftfeuchtigkeit, einen Luftdruck, einen Partikelkonzentrationswert und einen Luftqualitätswert.

10. Steuersystem nach einem der Ansprüche 1 bis 9, weiter eine Informationserlangungseinheit (150) umfassend, welche dazu konfiguriert ist, subjektspezifische historische Daten zu erhalten, welche einen zuvor bestimmten Parameterwert für das Subjekt beschreiben, und wobei die Steuereinheit (130) dazu konfiguriert ist, das Steuersignal weiter basierend auf den subjektspezifischen historischen Daten zu erzeugen.

11. Steuersystem nach Anspruch 10, wobei die subjektspezifischen historischen Daten zumindest eines der folgenden beinhalten:
historische HFNT-Betriebsparameter, welche mit dem Subjekt verknüpft sind; und
klinische Daten des Subjekts,
und wobei die klinischen Daten des Subjekts bevorzugt zumindest eines der folgenden beinhalten: ein Alter, eine Größe, ein Gewicht, einen BMI, Erkrankungen, Atemwegserkrankungen, eine Belastung durch Luftverschmutzung und eine Rauchervorgeschichte.

12. Steuersystem nach einem der Ansprüche 1 bis 11, wobei die Steuereinheit (130) dazu konfiguriert ist, das Steuersignal als Reaktion auf zumindest eines der folgenden zu erzeugen:
eine vorbestimmte Zeitspanne, welche seit Erzeugen eines vorherigen Steuersignals vergangen ist;
eine Änderung des erfassten physiologischen Parameterwerts, welche eine erste vorbestimmte Schwelle überschreitet; und
eine Änderung des erfassten Bewegungsparameterwerts, welche eine zweite vorbestimmte Schwelle überschreitet.

13. Computerprogramm, umfassend Computerprogrammcodemittel, welche dazu adaptiert sind, wenn das Computerprogramm auf einem Steuersystem nach Anspruch 1 läuft, ein Verfahren (200) zu implementieren, umfassend:
Erfassen (210) eines physiologischen Parameterwerts des Subjekts;
Erfassen (220) eines Bewegungsparameterwerts des Subjekts, welcher ein Aktivitätsniveau des Subjekts angibt;
Klassifizieren des Subjekts in eine von einer Vielzahl von Aktivitätsklassifizierungen basierend auf dem Bewegungsparameterwert; und
Erzeugen (230) eines Steuersignals für die HFNT-Vorrichtung basierend auf der Aktivitätsklassifizierung des Subjekts, dem physiologischen Parameterwert und dem Bewegungsparameterwert.

## Revendications

1. Système de commande (100) destiné à un dispositif de thérapie nasale à haut débit, HFNT, utilisé par un sujet, le système comprenant :
une unité de capteur physiologique (120) configurée pour détecter une valeur de paramètre physiologique du sujet ;
une unité de capteur de mouvement (122) configurée pour détecter une valeur de paramètre de mouvement du sujet indiquant un niveau d'activité du sujet ;
un composant de classification d'activité configuré pour classer le sujet dans l'une parmi une pluralité de classifications d'activité sur la base de la valeur de paramètre de mouvement ; et
une unité de commande (130) configurée pour générer un signal de commande pour le dispositif HFNT sur la base de la classification d'activité du sujet, de la valeur de paramètre physiologique et de la valeur de paramètre de mouvement.

2. Système de commande selon la revendication 1, dans lequel l'unité de commande (130) est configurée pour :
déterminer une valeur de paramètre de HFNT cible sur la base de la valeur de paramètre physiologique détectée et de la valeur de paramètre de mouvement ; et
générer le signal de commande sur la base de la valeur de paramètre de HFNT cible.

3. Système de commande selon la revendication 1 ou 2, comprenant en outre une unité d'interface de dispositif HFNT (140) configurée pour recevoir une valeur de paramètre de fonctionnement du dispositif HFNT, et dans lequel l'unité de commande (130) est configurée pour générer le signal de commande sur la base en outre de la valeur de paramètre de fonctionnement.

4. Système de commande selon l'une quelconque des revendications 1 à 3, comprenant en outre un dispositif portable (110), et dans lequel le dispositif portable comprend au moins l'une parmi l'unité de capteur physiologique (120) et l'unité de capteur de mouvement (124).

5. Système de commande selon l'une quelconque des revendications 1 à 4, dans lequel le signal de commande est configuré pour commander au moins l'un parmi un débit et une concentration en oxygène de l'air fourni par le dispositif HFNT.

6. Système de commande selon l'une quelconque des revendications 1 à 5, dans lequel le paramètre physiologique détecté inclut au moins l'une parmi :
une saturation en oxygène ;
une fréquence cardiaque ;
une variabilité de la fréquence cardiaque ;
une fréquence respiratoire ;
une variabilité de la fréquence respiratoire ;
une réponse cutanée galvanique ; et
une valeur de CO2 transcutanée.

7. Système de commande selon l'une quelconque des revendications 1 à 6, dans lequel le paramètre de mouvement détecté inclut au moins un parmi :
une accélération ;
une vitesse ;
un déplacement ;
une inclinaison ; et
un changement de position.

8. Système de commande selon l'une quelconque des revendications 1 à 7, comprenant en outre une unité de capteur environnemental (124) configurée pour détecter une valeur de paramètre d'environnement ambiant de l'environnement entourant le sujet, et dans lequel l'unité de commande (130) est configurée pour générer le signal de commande sur la base en outre de la valeur de paramètre ambiant.

9. Système de commande selon la revendication 8, dans lequel le paramètre d'environnement ambiant détecté inclut au moins une parmi : une température ; une humidité ; une pression de l'air ; une valeur de concentration de particules ; et une valeur de qualité de l'air.

10. Système de commande selon l'une quelconque des revendications 1 à 9, comprenant en outre une unité d'acquisition d'informations (150) configurée pour obtenir des données historiques spécifiques au sujet décrivant une valeur de paramètre précédemment déterminée pour le sujet, et dans lequel l'unité de commande (130) est configurée pour générer le signal de commande sur la base en outre des données historiques spécifiques au sujet.

11. Système de commande selon la revendication 10, dans lequel les données historiques spécifiques au sujet incluent au moins l'un parmi :
des paramètres historiques de fonctionnement du HFNT associés au sujet ; et
des données cliniques du sujet,
et de préférence dans lequel les données cliniques du sujet incluent au moins un parmi : un âge ; une taille ; un poids ; un IMC ; des affections médicales ; des affections respiratoires ; une exposition à la pollution de l'air ; et des antécédents de tabagisme.

12. Système de commande selon l'une quelconque des revendications 1 à 11, dans lequel l'unité de commande (130) est configurée pour générer le signal de commande en réponse à au moins un parmi :
une durée prédéterminée qui s'écoule depuis la génération d'un signal de commande précédent;
un changement dans la valeur de paramètre physiologique détectée qui dépasse un premier seuil prédéterminé ; et
un changement dans la valeur de paramètre de mouvement détectée qui dépasse un second seuil prédéterminé.

13. Programme informatique comprenant un moyen de code de programme informatique adapté, lorsque ledit programme informatique est exécuté sur un système de commande selon la revendication 1, pour mettre en œuvre un procédé (200) comprenant :
la détection (210) d'une valeur de paramètre physiologique du sujet ;
la détection (220) d'une valeur de paramètre de mouvement du sujet indiquant un niveau d'activité du sujet ;
le classement du sujet dans l'une parmi une pluralité de classifications d'activité sur la base de la valeur de paramètre de mouvement ; et
la génération (230) d'un signal de commande pour le dispositif HFNT sur la base de la classification d'activité du sujet, de la valeur de paramètre physiologique et de la valeur de paramètre de mouvement.
